# FASCICULE DE BREVET EUROPEEN

(11) **EP 0 665 005 B1**
(45) Date de publication et mention de la délivrance du brevet: **28.08.1996**
(21) Numéro de dépôt: 94402525.3
(22) Date de dépôt: 08.11.1994
(51) Int. Cl.: A61K 7/13

(54) **Composition de teinture d'oxydation des fibres kératiniques comprenant un dérivé de paraphénylènediamine et un dérivé de métaphénylènediamine, et procédé de teinture utilisant une telle composition**
Oxydative Haarfärbungszusammensetzung von einem Paraphenylendiamin und einem Metaphenylendiamin, und Verfahren zur Anwendung
Oxidative hair dye composition comprising a paraphenylene diamine and a metaphenylene diamine, and method of use

(30) Priorité: 24.01.1994 FR 9400700
(43) Date de publication de la demande: 02.08.1995
(73) Titulaire: L'OREAL, F-75008 Paris (FR)
(72) Inventeur: Audousset, Marie-Pascale, F-92600 Asnieres (FR); Cotteret, Jean, F-78480 Verneuil s/Seine (FR)
(74) Mandataire: Andral, Christophe André Louis

(56) Documents cités:
- EP-A- 0 007 537
- EP-A- 0 252 351
- GB-A- 2 085 483

## Description

La présente invention concerne une composition de teinture d'oxydation des fibres kératiniques et en particulier des fibres kératiniques humaines, comprenant en association, au moins une paraphénylènediamine substituée en position 2 sur le noyau benzénique et une métaphénylènediamine, dont les structures sont données ci-après dans la description. Elle concerne également l'utilisation d'une telle composition.

Il est connu de teindre les fibres kératiniques et en particulier les cheveux humains, avec des compositions de teinture contenant des précurseurs de colorants d'oxydation, en particulier des ortho- ou para- phénylènediamines, des ortho- ou para- aminophénols, généralement appelés "bases d'oxydation", et des coupleurs encore appelés modificateurs de coloration, plus particulièrement des métaphénylènediamines, des méta-aminophénols et des métadiphénols, qui permettent de modifier et d'enrichir de reflets les colorations "de fond" obtenues par les produits de condensation des bases d'oxydation.

On recherche activement dans le domaine de la teinture capillaire d'oxydation, des précurseurs de colorants d'oxydation et des coupleurs capables d'engendrer, lorsqu'ils sont associés, une coloration bleue, ayant une résistance satisfaisante à la lumière, aux lavages, aux intempéries, à la transpiration et aux différents traitements que peuvent subir les cheveux.

Jusqu'ici ces colorations ont été obtenues avec des teintures à base de paraphénylènediamine. Mais actuellement l'utilisation de la paraphénylène- diamine est remise en question pour des raisons toxicologiques.

Il est connu selon la demande EP-A-7 537 une composition tinctoriale comprenant , à titre de précurseur de colorant d'oxydation, des 2-hydroxyalkyle paraphénylènediamines. On connait également , d'après EP-A-252 351, des compositions tinctoriales comprenant, à titre de coupleurs, des 4-alcoxy-2-alkyl métaphénylènediamines.

Or, après d'importantes recherches menées sur la question, la Demanderesse vient maintenant de découvrir qu'il est possible d'obtenir de nouvelles teintures non toxiques, qui engendrent des colorations allant du bleu au bleu-pourpre, intenses et résistantes, en associant une paraphénylènediamine substituée en position 2 sur le noyau benzénique et une métaphénylènediamine, de structures respectivement définies ci-après.

Cette découverte est à la base de la présente invention.

La présente invention a ainsi pour objet une composition de teinture d'oxydation pour fibres kératiniques, en particulier pour fibres kératiniques humaines telles que les cheveux, du type comprenant, dans un milieu approprié pour la teinture, au moins un précurseur de colorant d'oxydation et au moins un coupleur, et caractérisée par le fait qu'elle contient, à titre de précurseur de colorant d'oxydation, au moins une paraphénylènediamine de formule (I) suivante : 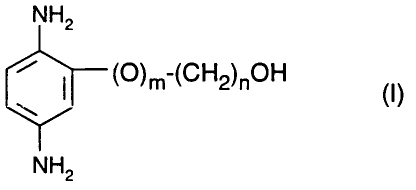 dans laquelle m est un nombre entier égal à zéro ou 1, et n est un nombre entier compris inclusivement entre 1 et 4,
et/ou au moins l'un des sels d'addition de cette paraphénylènediamine avec un acide,
et à titre de coupleur, au moins une métaphénylènediamine de formule (II) suivante : 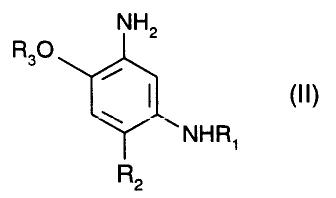 dans laquelle, R₁ désigne un atome d'hydrogène, un radical alkyle contenant de 1 à 3 atomes de carbone ou un radical monohydroxyalkyle contenant de 2 à 3 atomes de carbone, R₂ désigne un atome d'hydrogène, un radical mono- ou poly-hydroxyalcoxy contenant de 2 à 3 atomes de carbone et comportant de 1 à 2 groupements hydroxyle, R₃ désigne un radical alkyle contenant de 1 à 3 atomes de carbone, un radical mono- ou polyhydroxyalkyle contenant de 2 à 3 atomes de carbone et comportant de 1 à 2 groupements hydroxyle, un radical aminoalkyle contenant de 2 à 3 atomes de carbone, un groupement 2,4-diaminophénoxyalkyle dans lequel le radical alkyle contient de 1 à 4 atomes de carbone, étant entendu que lorsque R₁ désigne un atome d'hydrogène ou un radical alkyle, alors R₃ est différent d'un radical alkyle, et que lorsque R₂ désigne un radical mono- ou poly-hydroxyalcoxy, alors R₁ désigne obligatoirement un atome d'hydrogène et R₃ un radical mono- ou polyhydroxyalkyle,
et/ou au moins l'un des sels d'addition de cette métaphénylènediamine avec un acide.

Les nouvelles teintures ainsi obtenues permettent d'aboutir à des colorations allant du bleu au bleu-pourpre, soutenues, non toxiques et résistant particulièrement bien à la fois à la lumière, aux lavages, aux intempéries, à la transpiration et aux différents traitements que peuvent subir les cheveux. Elles sont tout particulièrement très résistantes aux shampooings.

Un autre objet de l'invention porte sur la composition prête à l'emploi, contenant les différents agents utilisés pour la teinture des fibres kératiniques définis ci-dessus et un agent oxydant.

L'invention vise également un procédé de teinture des fibres kératiniques, et en particulier des fibres kératiniques humaines telles que les cheveux, consistant à appliquer sur ces fibres au moins une composition (A) contenant, dans un milieu approprié pour la teinture, au moins un précurseur de colorant d'oxydation et un coupleur tels qu'ils ont été définis ci-avant, la couleur étant révélée à pH alcalin neutre ou acide à l'aide d'un agent oxydant qui est ajouté juste au moment de l'emploi à la composition (A) ou qui est présent dans une composition (B) appliquée simultanément ou séquentiellement de façon séparée.

L'invention a également pour objet des dispositifs de teinture ou "kits" à plusieurs compartiments, dont le premier compartiment contient au moins une paraphénylènediamine de formule (I), à titre de précurseur de colorant d'oxydation, et au moins un coupleur de formule (II), et le deuxième compartiment un agent oxydant.

D'autres caractéristiques, aspects, objets et avantages de l'invention apparaîtront encore plus clairement à la lecture de la description et des exemples qui suivent.

Les sels d'acide qui peuvent être utilisés selon l'invention sont choisis de préférence parmi les chlorhydrates, les sulfates, les bromhydrates et les tartrates.

Parmi les précurseurs de colorant d'oxydation de formule (I) utilisables dans le cadre de la présente invention, on préfère mettre en oeuvre les composés suivants : la 2-(β-hydroxyméthyl) paraphénylènediamine, la 2-(β-hydroxyéthyl) paraphénylènediamine, et la 2-(β-hydroxyéthyloxy) paraphénylènediamine.

La concentration en ce(s) précurseurs(s) de formule (I) ou leurs sels peut varier entre 0,01 et 10 % en poids environ par rapport au poids total de la composition de teinture et de préférence entre 0,05 et 5 % en poids environ.

Parmi les coupleurs de formule (II), on préfère plus particulièrement mettre en oeuvre le 1-(β-hydroxyéthyloxy) 2,4-diamino benzène, le 1,3-bis-(2,4-diaminophénoxy) propane, le 1,3-bis-(2,4-diaminophénoxy) méthane, le 1-méthoxy 2-amino 4-(β-hydroxyéthylamino) benzène, le 1-(β-aminoéthyloxy) 2,4-diaminobenzène, le 1-(β-hydroxyéthyloxy) 2-amino 4-méthylamino benzène, le 1,3-diamino 4,6-bis-(β-hydroxyéthyloxy) benzène, le 1-(β,γ-dihydroxypropyloxy) 2,4-diamino benzène.

La concentration en coupleur(s) de formule (II) ou leurs sels peut varier entre 0,001 et 3% en poids environ par rapport au poids total de la composition de teinture et de préférence entre 0,05 et 2 % en poids environ.

Des compositions de teinture d'oxydation plus particulièrement préférées selon l'invention comprennent à titre de précurseur de colorant d'oxydation la 2-(β-hydroxyéthyl) paraphénylènediamine ou l'un de ses sels, et à titre de coupleur le 1-(β-hydroxyéthyloxy) 2,4-diamino benzène ou le 1-méthoxy 2-amino 4-(β-hydroxyéthylamino) benzène ou l'un de leurs sels.

D'autres compositions, également particulièrement intéressantes, comprennent à titre de précurseur de colorant d'oxydation la 2-(β-hydroxyéthyloxy) paraphénylènediamine ou l'un de ses sels, et à titre de coupleur, le 1-(β-hydroxyéthyloxy) 2,4-diamino benzène ou le 1-méthoxy 2-amino 4-(β-hydroxyéthylamino) benzène ou l'un de leurs sels.

L'agent oxydant est choisi de préférence parmi le peroxyde d'hydrogène, le peroxyde d'urée, les bromates de métaux alcalins, les persels tels que les perborates et les persulfates. L'utilisation du peroxyde d'hydrogène est particulièrement préférée.

La composition (A), qui renferme l'association des colorants telle que décrite ci-dessus, peut avoir un pH compris entre 3 et 11, qui peut être ajusté à la valeur choisie au moyen soit d'agents alcalinisants habituellement utilisés en teinture des fibres kératiniques, tels que l'ammoniaque, les carbonates alcalins, les alcanolamines telles que par exemple les mono-, di - et triéthanolamines ainsi que leurs dérivés, les hydroxydes de sodium ou de potassium, les composés de formule : 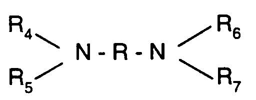 dans laquelle R est un reste propylène éventuellement substitué par un groupement hydroxyle ou un radical alkyle en C₁-C₄ ; R₄ R₅, R₆ et R₇, simultanément ou indépendamment l'un de l'autre, représentent un atome d'hydrogène, un radical alkyle en C₁-C₄ ou hydroxyalkyle en C₁-C₄,
soit au moyen d'agents acidifiants classiques, tels que les acides minéraux ou organiques comme par exemple les acides chlorhydrique, tartrique, citrique et phosphorique.

Le pH de la composition (B) renfermant l'agent oxydant tel que défini ci-dessus est tel qu'après mélange avec la composition (A), le pH de la composition résultante appliquée sur les fibres kératiniques humaines varie de préférence entre 3 et 11. Il est ajusté à la valeur désirée à l'aide d'agents acidifiants ou éventuellement alcalinisants bien connus de l'état de la technique, tels que ceux décrits ci-dessus.

La composition oxydante (B) est de préférence constituée par une solution d'eau oxygénée.

Selon un mode de réalisation préféré du procédé de teinture de l'invention, on mélange, au moment de l'emploi, la composition de teinture (A) décrite ci-dessus avec une solution oxydante en une quantité suffisante pour développer une coloration. Le mélange obtenu est ensuite appliqué sur les fibres kératiniques humaines et on laisse poser pendant 5 à 40 minutes, de préférence 15 à 30 minutes, après quoi on rince, on lave au shampooing, on rince à nouveau et on sèche.

Les compositions de teinture peuvent encore contenir, en plus des colorants définis ci-dessus, d'autres coupleurs et/ou des colorants directs, notamment pour modifier les nuances ou les enrichir en reflets.

Les compositions de teinture peuvent également contenir des agents antioxydants. Ceux-ci peuvent être choisis en particulier parmi le sulfite de sodium, l'acide thioglycolique, l'acide thiolactique, le bisulfite de sodium, l'acide déhydroascorbique, l'hydroquinone, la 2-méthyl hydroquinone, la tertiobutyl hydroquinone et l'acide homogentisique et sont alors généralement présents dans des proportions comprises entre environ 0,05 et 1,5 % en poids par rapport au poids total de la composition.

Les compositions de teinture contiennent également, dans leur forme de réalisation préférée, des agents tensioactifs bien connus de la technique, dans des proportions comprises entre environ 0,5 et 55 % en poids, et de préférence entre 2 et 50 % en poids par rapport au poids total de la composition, des solvants organiques, dans des proportions comprises entre environ 1 et 40 % en poids, et en particulier entre 5 et 30 % en poids par rapport au poids total de la composition, ou tout autre adjuvant cosmétiquement acceptable et connu de la technique antérieure en teinture d'oxydation capillaire.

La composition appliquée sur les cheveux peut se présenter sous des formes diverses, telles que sous forme de liquide, de crème, de gel, ou sous toute autre forme appropriée pour réaliser une teinture des fibres kératiniques, et notamment des cheveux humains. En particulier, elle peut être conditionnée sous pression en flacon aérosol en présence d'un agent propulseur et former une mousse.

Des exemples concrets illustrant l'invention vont maintenant être donnés. On commencera par définir les tests qui ont été utilisés pour évaluer les performances des teintures d'oxydation selon l'invention, concernant leur résistance à la transpiration, à la lumière et aux shampooings, aux intempéries ou à la permanente.

### Résistance à la transpiration :

On utilise une solution de sueur synthétique de composition suivante : 10 g de NaCl, 1 g d'hydrogénophosphate de potassium, 0,25 g d'histidine, de l'acide lactique jusqu'à pH = 3,2 et de l'eau distillée pour compléter à 100 g.

Dans un cristallisoir recouvert d'un verre de montre et renfermant cette solution de sueur, on immerge les mèches de cheveux teints qu'on laisse séjourner de 20 à 50 heures à 37° C. On rince ensuite les mèches et on les sèche.

### Résistance à la lumière (Xenotest) :

Les cheveux teints sont fixés sur un support (carton ou plastique). Ces supports sont disposés sur des porte-échantillons qui tournent autour d'une lampe Xénon pendant une durée variant de 20 à 80 heures sous un taux d'humidité variant de 25 à 75% HR (Humidité Relative) et à une température de 25° C.

### Résistance aux shampooings (machine Ahiba-Texomat) :

On place des mèches de cheveux teints dans un panier que l'on immerge dans une solution d'un shampooing standard. Le panier est soumis à un mouvement de va-et-vient vertical de fréquence variable ainsi qu'à un mouvement de rotation qui reproduisent l'action d'un frottement manuel, ce qui engendre la formation de mousse.
Après 3 minutes d'épreuve, on retire les mèches que l'on rince puis sèche. Les mèches teintes peuvent être soumises à plusieurs épreuves shampooings consécutives.

### Résistance aux intempéries (Epreuve combinée) :

Les mèches teintes sont exposées à la lumière forte ( Xenotest 40 h ), sous une humidité relative de 60 %, et simultanément, toutes les 12 heures, et pendant une durée de 20 minutes, elles sont soumises à une aspersion d'eau.

### Résistance à la permanente :

Les mèches teintes sont immergées dans une solution réductrice de permanente Dulcia Vital (L'Oréal), de force variant de 1 à 3, pendant une durée variant de 10 à 20 minutes ; les mèches sont rincées ; on les trempe ensuite dans une solution de fixateur (oxydant), pendant 5 minutes. Après rinçage à l'eau, lavage au shampooing standard et rinçage à l'eau, on les sèche.

### EXEMPLE 1 :

On a préparé la composition de teinture, conforme à l'invention, suivante :

| | |
|---|---|
| - 2-(β-hydroxyéthyl) paraphénylènediamine, dichlorhydrate | 0,675 g |
| - 1-(β-hydroxyéthyloxy) 2,4-diamino benzène | 0,672 g |
| - Alcool oléique polyglycérolé à 2 moles de glycérol | 4,0 g |
| - Alcool oléique polyglycérolé à 4 moles de glycérol (78 % de MA) | 5,7 g MA |
| - Acide oléique | 3,0 g |
| - Amine oléique à 2 moles d'oxyde d'éthylène, vendue sous la dénomination Ethomeen 012 par la Société AKZO | 7,0 g |
| - Laurylaminosuccinamate de diéthylaminopropyle, sel de sodium à 55% de MA | 3,0 g MA |
| - Alcool oléique | 5,0 g |
| - Diéthanolamide d'acide oléique | 12,0 g |
| - Propylèneglycol | 3,5 g |
| - Alcool éthylique | 7,0 g |
| - Dipropylèneglycol | 0,5 g |
| - Monométhyléther de propylèneglycol | 9,0 g |
| - Métabisulfite de sodium en solution aqueuse à 35% de MA | 0,4 g MA |
| - Acétate d'ammonium | 0,8 g |
| - Antioxydant, séquestrant, | qs |
| - Parfum, conservateur, | qs |
| - Ammoniaque à 20 % de NH₃ | 10,0 g |
| - Eau déminéralisée qsp | 100,0 g |

Au moment de l'emploi, on a mélangé cette composition poids pour poids avec de l'eau oxygénée titrant 20 volumes (6% en poids), de pH 3.
On a obtenu un mélange de pH 9,8.

On a ensuite appliqué ce mélange sur des cheveux gris à 90% de blancs, pendant 30 minutes. Après rinçage, lavage au shampooing, rinçage et séchage, les cheveux sont teints dans une nuance d'un bleu puissant qui résiste en particulier remarquablement bien aux shampooings.

### EXEMPLE 2

On a préparé la composition de teinture, conforme l'invention, suivante :

| | |
|---|---|
| - 2-(β-hydroxyéthyloxy) paraphénylènediamine, dichlorhydrate | 0,723 g |
| - 1-méthoxy 2-amino 4-(β-hydroxyéthylamino) benzène | 0,759 g |
| - Alcool oléique polyglycérolé à 2 moles de glycérol | 4,0 g |
| - Alcool oléique polyglycérolé à 4 moles de glycérol (78 % de MA) | 5,7 g MA |
| - Acide oléique | 3,0 g |
| - Amine oléique à 2 moles d'oxyde d'éthylène, vendue sous la dénomination Ethomeen 012 par la Société AKZO | 7,0 g |
| - Laurylaminosuccinamate de diéthylaminopropyle, sel de sodium à 55% de MA | 3,0 g MA |
| - Alcool oléique | 5,0 g |
| - Diéthanolamide d'acide oléique | 12,0 g |
| - Propylèneglycol | 3,5 g |
| - Alcool éthylique | 7,0 g |
| - Dipropylèneglycol | 0,5 g |
| - Monométhyléther de propylèneglycol | 9,0 g |
| - Métabisulfite de sodium en solution aqueuse à 35% de MA | 0,4 g MA |
| - Acétate d'ammonium | 0,8 g |
| - Antioxydant, séquestrant, | qs |
| - Parfum, conservateur, | qs |
| - Ammoniaque à 20% de NH₃ | 10,0 g |
| - Eau déminéralisée qsp. | 100 g |

Au moment de l'emploi, on a mélangé cette composition poids pour poids avec de l'eau oxygénée titrant 20 volumes (6% en poids), de pH 3.

On a obtenu un mélange de pH 9,8.

On a ensuite appliqué ce mélange sur des cheveux gris à 90% de blancs, pendant 30 minutes. Après rinçage, lavage au shampooing, rinçage et séchage, les cheveux sont teints dans une nuance d'un bleu soutenu qui résiste en particulier remarquablement bien aux shampooings.

## Revendications

1. Composition de teinture d'oxydation pour fibres kératiniques, en particulier pour fibres kératiniques humaines telles que les cheveux, du type comprenant dans un milieu approprié pour la teinture, au moins un précurseur de colorant d'oxydation et au moins un coupleur, caractérisée par le fait qu'elle contient, à titre de précurseur de colorant d'oxydation, au moins une paraphénylènediamine de formule (I) suivante : 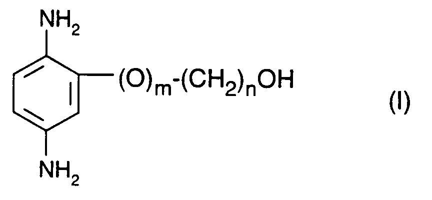 dans laquelle m est un nombre entier égal à zéro ou 1, et n est un nombre entier compris inclusivement entre 1 et 4,
et/ou au moins l'un des sels d'addition de cette paraphénylènediamine avec un acide,
et à titre de coupleur, au moins une métaphénylènediamine de formule (II) suivante : 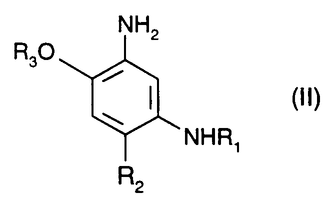 dans laquelle, R₁ désigne un atome d'hydrogène, un radical alkyle contenant de 1 à 3 atomes de carbone, ou un radical monohydroxyalkyle contenant de 2 à 3 atomes de carbone, R₂ désigne un atome d'hydrogène, un radical mono-ou poly-hydroxyalcoxy contenant de 2 à 3 atomes de carbone et comportant de 1 à 2 groupements hydroxyle, R₃ désigne un radical alkyle contenant de 1 à 3 atomes de carbone, un radical mono- ou polyhydroxyalkyle contenant de 2 à 3 atomes de carbone et comportant de 1 à 2 groupements hydroxyle, un radical aminoalkyle contenant de 2 à 3 atomes de carbone, un groupement 2,4-diaminophénoxyalkyle dans lequel le radical alkyle contient de 1 à 4 atomes de carbone, étant entendu que lorsque R₁ désigne un atome d'hydrogène ou un radical alkyle, alors R₃ est différent d'un radical alkyle, et que lorsque R₂ désigne un radical mono- ou poly-hydroxyalcoxy, alors R₁ désigne obligatoirement un atome d'hydrogène et R₃ un radical mono- ou polyhydroxyalkyle,
et/ou au moins l'un des sels d'addition de cette métaphénylènediamine avec un acide.

2. Composition de teinture selon la revendication 1, caractérisée par le fait que la paraphénylènediamine de formule (I) est choisie parmi la 2-(β-hydroxyméthyl) paraphénylènediamine, la 2-(β-hydroxyéthyl) paraphénylènediamine, la 2-(β-hydroxyéthyloxy) paraphénylènediamine, et leurs sels d'addition avec un acide.

3. Composition de teinture selon la revendication 1 ou 2, caractérisée par le fait que le coupleur de formule (II) est choisi parmi le 1-(β-hydroxyéthyloxy) 2,4-diamino benzène, le 1,3-bis-(2,4-diaminophénoxy) propane, le 1,3-bis-(2,4-diaminophénoxy) méthane, le 1-méthoxy 2-amino 4-(β-hydroxyéthylamino) benzène, le 1-(β-aminoéthyloxy) 2,4-diaminobenzène, le 1-(β-hydroxyéthyloxy) 2-amino 4-méthylamino benzène, le 1,3-diamino 4,6-bis-(β-hydroxyéthyloxy) benzène, le 1-(β,γ-dihydroxypropyloxy) 2,4-diamino benzène.

4. Composition de teinture selon l'une quelconque des revendications 1 à 3, caractérisée par le fait qu'elle contient au moins la 2-(β-hydroxyéthyl) paraphénylènediamine à titre de précurseur de colorant d'oxydation de formule (I) et au moins le 1-(β-hydroxyéthyloxy) 2,4-diamino benzène ou le 1-méthoxy 2-amino 4-(β-hydroxyéthylamino) benzène à titre de coupleur, ou l'un de leurs sels d'addition avec un acide.

5. Composition de teinture selon l'une quelconque des revendications 1 à 3, caractérisée par le fait qu'elle contient au moins la 2-(β-hydroxyéthyloxy) paraphénylènediamine ou l'un de ses sels, à titre de précurseur de colorant d'oxydation de formule (I) et au moins le 1-(β-hydroxyéthyloxy) 2,4-diamino benzène ou le 1-méthoxy 2-amino 4-(β-hydroxyéthylamino) benzène ou l'un de leurs sels d'addition avec un acide, à titre de coupleur.

6. Composition de teinture selon l'une quelconque des revendications 1 à 5, caractérisée par le fait que les sels d'addition avec un acide sont choisis parmi les chlorhydrates, les sulfates, les bromhydrates et les tartrates.

7. Composition de teinture selon l'une quelconque des revendications 1 à 6, caractérisée par le fait que la paraphénylènediamine de formule (I), ou ses sels, est présente dans une concentration comprise entre 0,01 et 10 % en poids par rapport au poids total de la composition, et de préférence entre 0,05 et 5 % en poids, et que le coupieur de formule (II), ou ses ses, est présent dans une concentration comprise entre 0,001 et 3 % en poids et de préférence entre 0,05 et 2 % par rapport au poids total de la composition.

8. Composition de teinture selon l'une quelconque des revendications 1 à 7, prête à l'emploi, caractérisée en ce qu'elle contient en outre un agent oxydant et qu'elle possède un pH compris entre 3 et 11.

9. Procédé de teinture des fibres kératiniques et en particulier des fibres kératiniques humaines telles que les cheveux, caractérisé par le fait qu'il consiste à appliquer sur les fibres une composition de teinture (A) selon l'une quelconque des revendications 1 à 8, et à révéler la couleur en milieu alcalin neutre ou acide à l'aide d'un agent oxydant qui est ajouté juste au moment de l'emploi à cette composition (A) ou qui est présent dans une composition (B) appliquée simultanément ou séquentiellement de façon séparée.

10. Dispositif à plusieurs compartiments ou "Kit" pour la teinture des fibres kératiniques et en particulier des fibres kératiniques humaines telles que les cheveux, caractérisé par le fait qu'il comporte au moins deux compartiments, dont l'un d'entre eux renferme une composition (A) telle que définie dans l'une quelconque des revendications 1 à 8, et un autre une composition (B) comprenant un agent oxydant dans un milieu approprié pour la teinture.

11. Utilisation d'une composition de teinture selon l'une quelconque des revendications 1 à 8 ou d'un dispositif de teinture ou "Kit" à plusieurs compartiments selon la revendication 10, pour la teinture des fibres kératiniques humaines telles que les cheveux.

## Claims

1. A composition for the oxidation dyeing of keratin fibres, in particular for human keratin fibres such as hair, of the type comprising, in a medium which is suitable for dyeing, at least one oxidation dye precursor and at least one coupling agent, and which composition is characterized in that it contains, as oxidation dye precursor, at least one para-phenylenediamine of following formula (I): 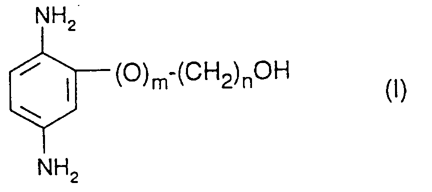 in which m is an integer equal to zero or 1 and n is an integer between 1 and 4 inclusively,
and/or at least one of the addition salts of this para-phenylenediamine with an acid,
and, as coupling agent, at least one meta-phenylenediamine of following formula (II): 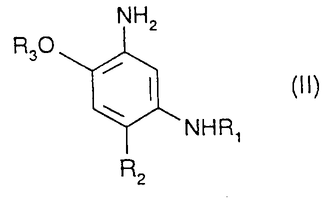 in which R₁ denotes a hydrogen atom, an alkyl radical containing from 1 to 3 carbon atoms or a monohydroxyalkyl radical containing from 2 to 3 carbon atoms, R₂ denotes a hydrogen atom, a mono- or polyhydroxyalkoxy radical containing from 2 to 3 carbon atoms and composed of 1 to 2 hydroxyl groups, R₃ denotes an alkyl radical containing from 1 to 3 carbon atoms, a mono- or polyhydroxyalkyl radical containing from 2 to 3 carbon atoms and composed of 1 to 2 hydroxyl groups, an aminoalkyl radical containing from 2 to 3 carbon atoms, a 2,4-diaminophenoxyalkyl group in which the alkyl radical contains from 1 to 4 carbon atoms, it being understood that, when R₁ denotes a hydrogen atom or an alkyl radical, R₃ is then other than an alkyl radical, and, when R₂ denotes a mono- or polyhydroxyalkoxy radical, R₁ then necessarily denotes a hydrogen atom and R₃ denotes a mono- or polyhydroxyalkyl radical, and/or at least one of the addition salts of this meta-phenylenediamine with an acid.

2. Dye composition according to Claim 1, characterized in that the para-phenylenediamine of formula (I) is chosen from 2-(β-hydroxymethyl)-para-phenylenediamine, 2-(β-hydroxyethyl)-para-phenylenediamine, 2-(β-hydroxyethyloxy)-para-phenylenediamine and the addition salts thereof with an acid.

3. Dye composition according to Claim 1 or 2, characterized in that the coupling agent of formula (II) is chosen from 1-(β-hydroxyethyloxy)-2,4-diaminobenzene, 1,3-bis(2,4-diaminophenoxy)propane, 1,3-bis(2,4-diaminophenoxy)methane, 1-methoxy-2-amino-4-(β-hydroxyethylamino)benzene, 1-(β-aminoethyloxy)-2,4-diaminobenzene, 1-(β-hydroxyethyloxy)-2-amino-4-methylaminobenzene, 1,3-diamino-4,6-bis(β-hydroxyethyloxy)benzene and 1-(β,γ-dihydroxypropyloxy)-2,4-diaminobenzene.

4. Dye composition according to any one of Claims 1 to 3, characterized in that it contains, as oxidation dye precursor of formula (I), at least 2-(β-hydroxyethyl)-para-phenylenediamine and, as coupling agent, at least 1-(β-hydroxyethyloxy)-2,4-diaminobenzene or 1-methoxy-2-amino-4-(β-hydroxyethylamino)benzene, or one of the addition salts thereof with an acid.

5. Dye composition according to any one of Claims 1 to 3, characterized in that it contains, as oxidation dye precursor of formula (I), at least 2-(β-hydroxyethyloxy)-para-phenylenediamine and, as coupling agent, at least 1-(β-hydroxyethyloxy)-2,4-diaminobenzene or 1-methoxy-2-amino-4-(β-hydroxyethylamino)benzene, or one of the addition salts thereof with an acid.

6. Dye composition according to any one of Claims 1 to 5, characterized in that the addition salts with an acid are chosen from hydrochlorides, sulphates, hydrobromides and tartrates.

7. Dye composition according to any one of Claims 1 to 6, characterized in that the para-phenylenediamine of formula (I), or the salts thereof, is present in a concentration of between 0.01 and 10 % by weight relative to the total weight of the composition, and preferably between 0.05 and 5 % by weight, and that the coupling agent of formula (II), or the salts thereof, is present in a concentration of between 0.001 and 3 % by weight, and preferably between 0.05 and 2 %, relative to the total weight of the composition.

8. Dye composition according to any one of Claims 1 to 7, which is a ready-to-use composition, characterized in that it additionally contains an oxidizing agent and in that it has a pH of between 3 and 11.

9. Process for dyeing keratin fibres and in particular human keratin fibres such as hair, characterized in that it consists in applying to the fibres a dye composition (A) according to any one of Claims 1 to 8, and in developing the colour in an alkaline, neutral or acidic medium using an oxidizing agent which is added only at the time of use to this composition (A), or which is present in a composition (B) that is applied simultaneously or sequentially in a separate manner.

10. Device containing several compartments, or "kit" for dyeing keratin fibres, and in particular human keratin fibres such as hair, characterized in that it is composed of at least two compartments, one of which contains a composition (A) as defined in any one of Claims 1 to 8, and another of which contains a composition (B) comprising an oxidizing agent in a medium which is suitable for dyeing.

11. Use of a dye composition according to any one of Claims 1 to 8, or of a dyeing device or "kit" containing several compartments according to Claim 10, for dyeing human keratin fibres such as hair.

## Patentansprüche

1. Zusammensetzung zum oxidativen Färben von Keratinfasern, insbesondere von menschlichen Keratinfasern, wie dem Haar, die in einem zum Färben geeigneten Medium mindestens ein Farbstoffvorprodukt eines Oxidationsfarbstoffs und mindestens einen Kuppler enthält, dadurch gekennzeichnet, daß
sie als Farbstoffvorprodukt des Oxidationsfarbstoffs mindestens ein p-Phenylendiamin der folgenden Formel (I) 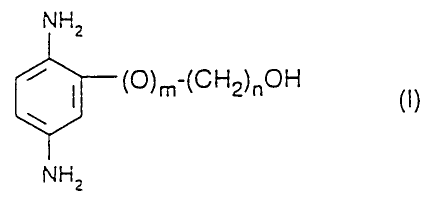 worin bedeuten:
m Null oder 1 und
n eine ganze Zahl von 1 bis 4,
und/oder mindestens ein Additionssalz dieses p-Phenylendiamins mit einer Säure,
und als Kuppler mindestens ein m-Phenylendiamin der folgenden Formel (II) 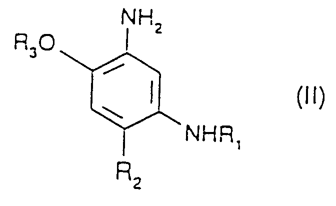 worin dedeuten:
R₁ Wasserstoff, eine Alkylgruppe mit 1 bis 3 Kohlenstoffatomen oder eine Monohydroxyalkylgruppe mit 2 bis 3 Kohlenstoffatomen,
R₂ Wasserstoff, eine Mono- oder Polyhydroxyalkoxygruppe mit 2 bis 3 Kohlenstoffatomen und mit 1 bis 2 Hydroxygruppen und
R₃ eine Alkylgruppe mit 1 bis 3 Kohlenstoffatomen, eine Mono- oder Polyhydroxyalkylgruppe mit 2 bis 3 Kohlenstoffatomen und mit 1 bis 2 Hydroxygruppen, eine Aminoalkylgruppe mit 2 bis 3 Kohlenstoffatomen, eine 2,4-Diaminophenoxyalkylgruppe, wobei die Alkylgruppe 1 bis 4 Kohlenstoffatome enthält,
mit der Maßgabe, daß R₃ keine Alkylgruppe bedeutet, wenn R₁ ein Wasserstoffatom oder eine Alkylgruppe bedeutet, und R₁ ein Wasserstoffatom und R₃ eine Mono- oder Polyhydroxyalkylgruppe bedeuten muß, wenn R₂ eine Mono- oder Polyhydroxyalkoxygruppe bedeutet, und/oder mindestens ein Additionssalz dieses m-Phenylendiamins mit einer Säure enthält.

2. Zusammensetzung zum Färben nach Anspruch 1, dadurch gekennzeichnet, daß das p-Phenylendiamin der Formel (I) unter 2-Hydroxymethyl-p-phenylendiamin, 2-(β-Hydroxyethyl)-p-phenylendiamin, 2-(β-Hydroxyethyloxy)-p-phenylendiamin oder den Additionssalzen dieser Verbindungen mit einer Säure ausgewählt ist.

3. Zusammensetzung zum Färben nach Anspruch 1 oder 2, dadurch gekennzeichnet, daß der Kuppler der Formel (II) unter 1-(β-Hydroxyethyloxy)-2,4-diaminobenzol, 1,3-Bis(2,4-diaminophenoxy)propan, Bis(2,4-diaminophenoxy)methan, 1-Methoxy-2-amino-4-(β-hydroxyethylamino)benzol, 1-(β-Aminoethyloxy)-2,4-diaminobenzol, 1-(β-Hydroxyethyloxy)-2-amino-4-methylaminobenzol, 1,3-Diamino-4,6-bis(β-hydroxyethyloxy)benzol und 1-(β,γ-Dihydroxypropyloxy)-2,4-diaminobenzol ausgewählt ist.

4. Zusammensetzung zum Färben nach einem der Ansprüche 1 bis 3, dadurch gekennzeichnet, daß sie mindestens 2-(β-Hydroxyethyl)-p-phenylendiamin als Farbstoffvorprodukt des Oxidationsfarbstoffs der Formel (I) und mindestens 1-(β-Hydroxyethyloxy)-2,4-diaminobenzol oder 1-Methoxy-2-amino-4-(β-hydroxyethylamino)benzol als Kuppler oder eines der Additionssalze dieser Verbindungen mit einer Säure enthält.

5. Zusammensetzung zum Färben nach einem der Ansprüche 1 bis 3, dadurch gekennzeichnet, daß sie mindestens 2-(β-Hydroxyethyloxy)-p-phenylendiamin oder eines seiner Salze als Farbstoffvorprodukt des Oxidationsfarbstoffs der Formel(I) und als Kuppler mindestens 1-(β-Hydroxyethyloxy)-2,4-diaminobenzol oder 1-Methoxy-2-amino-4-(β-hydroxyethylamino)benzol oder eines der Additionssalze dieser Verbindungen mit einer Säure enthält.

6. Zusammensetzung zum Färben nach einem der Ansprüche 1 bis 5, dadurch gekennzeichnet, daß die Additionssalze mit einer Säure unter den Hydrochloriden, Sulfaten, Hydrobromiden und Tartraten ausgewählt sind.

7. Zusammensetzung zum Färben nach einem der Ansprüche 1 bis 6, dadurch gekennzeichnet, daß das p-Phenylendiamin der Formel (I) oder seine Salze in einer Konzentration von 0,01 bis 10 Gew.-% und vorzugsweise von 0,05 bis 5 Gew.-%, bezogen auf das Gesamtgewicht der Zusammensetzung, und der Kuppler der Formel (II) oder seine Salze in einer Konzentration von 0,001 bis 3 Gew.-% und vorzugsweise 0,05 bis 2 Gew.-%, bezogen auf das Gesamtgewicht der Zusammensetzung, vorliegen.

8. Zusammensetzung zum Färben nach einem der Ansprüche 1 bis 7, die anwendungsfertig ist und die dadurch gekennzeichnet ist, daß sie ferner ein Oxidationsmittel enthält und einen pH-Wert von 3 bis 11 aufweist.

9. Verfahren zum Färben von Keratinfasern und insbesondere von menschlichen Keratinfasern, wie dem Haar, dadurch gekennzeichnet, daß es darin besteht, auf die Fasern eine Zusammensetzung zum Färben (A) nach einem der Ansprüche 1 bis 8 aufzutragen und die Farbe im alkalischen, neutralen oder sauren Medium mit einem Oxidationsmittel zu entwickeln, das dieser Zusammensetzung (A) bei der Anwendung zugesetzt wird oder das in einer Zusammensetzung (B) vorliegt, die gleichzeitig oder getrennt davon anschließend aufgetragen wird.

10. Vorrichtung mit mehreren Anteilungen oder "Kit" zum Färben von Keratinfasern und insbesondere menschlichen Keratinfasern, wie dem Haar, dadurch gekennzeichnet, daß sie mindestens zwei Abteilungen aufweist, wobei eine Abteilung eine Zusammensetzung (A) nach einem der Ansprüche 1 bis 8 und die andere eine Zusammensetzung (B) enthält, die in einem zum Färben geeigneten Medium ein Oxidationsmittel enthält.

11. Verwendung einer Zusammensetzung zum Färben nach einem der Ansprüche 1 bis 8 oder einer Vorrichtung zum Färben oder eines "Kits" mit mehreren Abteilungen nach Anspruch 10 zum Färben von menschlichen Keratinfasern, wie dem Haar.
